# EUROPEAN PATENT APPLICATION

(11) **EP 3 882 321 A1**
(43) Date of publication of application: **22.09.2021**
(21) Application number: 20305279.0
(22) Date of filing: 17.03.2020
(51) Int. Cl.: C09D 11/52, C09D 11/322

(54) **METHOD OF PREPARATION OF A GOLD ELECTRODE**

(71) Applicant: Valotec, 94800 Villejuif (FR); Université de Paris, 75006 Paris (FR); Centre National de la Recherche Scientifique, 75016 Paris (FR)
(72) Inventor: CAPITAO, Dany, 91260 Juvisy-sur-Orge (FR); MAURIN, Antoine, 77600 Bussy Saint-Georges (FR); MEKHMOUKHEN, Samia, 75019 Paris (FR); NOEL, Vincent, 95300 Pontoise (DE)
(74) Representative: Icosa

(57) **Abstract**

The present invention relates to a method of preparation of a gold electrode comprising the steps of:
a) inkjet printing on a substrate an ink comprising gold nanoparticles functionalized with a compound Cap of formula (I)
HS-A-Polyalkylene glycol (I)
wherein A is a bound or an alkyl chain comprising 1 to 12 carbon atoms;
wherein polyalkylene glycol is polyethylene glycol, polypropylene glycol or a mixture thereof; and

b) annealing printed ink;
wherein the compound Cap has an average molar mass of less than 500 g/mol, preferably an average molar mass of about 200 g/mol.

The present invention also relates to an electrode obtainable by the method of the invention, a sensor comprising said electrode and the use of said sensor.

## Description

### FIELD OF INVENTION

The present invention pertains to the field of metallic electrodes. In particular, the invention relates to a method of preparation of a gold electrode and electrodes obtainable by said method.

### BACKGROUND OF INVENTION

One step to manufacture a sensor is to deposit conductive electrodes on a substrate with a continuous conductive path. In the case of miniature sensors, said conductive path must be as fine and as precise as possible. Conductive electrodes, such as gold electrodes, have been typically prepared by evaporation. However, this technique often results in the appearance of cracks in the conductive path, which gives poor conduction results. One way to overcome this problem is to deposit many gold layers, which is not compatible with obtaining a fine and precise conductive path. It is also very difficult to obtain very fine conductive paths with the evaporation technique due to the use of a shadow mask to delimit the deposit area.

Recently, inkjet printing has been investigated to deposit materials on substrates. Among tremendous advantages, inkjet printing represents a less expensive and easier to implement process than evaporation and can provide a fine and precise pattern while limiting the loss of material compared to the evaporation technique.

Gold nanoparticles are formulated in an ink, comprising a solvent, inkjet printed on a substrate, then the solvent is evaporated. To ensure a good dispersion of the gold nanoparticles in the ink, an organic capping agent on said nanoparticles has to be used. However, to obtain a continuous conductive path, physical contact between gold nanoparticles is needed. Thus, the capping agent has to be removed. After solvent evaporation, a thermal treatment is then required to remove the capping agent and ensure the obtention of a conductive electrode. A high temperature treatment is often used as it allows for the complete removal of the organic capping agent.

However, the temperature of the thermal treatment is limited by the thermal resistance of the substrate, especially in the case of organic substrates. Indeed, a high temperature is not compatible with organic substrates, as such a thermal treatment would cause the degradation of the substrate itself.

Thus, there is thus a need for a method of preparation of gold electrodes compatible with all substrates, inorganic or organic substrates, at low temperature.

The Applicant found that a method comprising inkjet printing on a substrate an ink comprising gold nanoparticles functionalized with capping agent comprising a mercaptopolyalkylene glycol having an average molar mass of less than 500 g/mol allowed for low-temperature preparation of gold electrodes.

### SUMMARY

The present invention relates to a method of preparation of a gold electrode comprising the steps of:
a) inkjet printing on a substrate an ink comprising gold nanoparticles functionalized with a compound Cap of formula (I)

   HS-A-Polyalkylene glycol (I)

   wherein A is a bound or an alkyl chain comprising 1 to 12 carbon atoms;
   wherein polyalkylene glycol is polyethylene glycol, polypropylene glycol, or a mixture thereof; and
b) annealing printed ink;
wherein the compound Cap has an average molar mass of less than 500 g/mol, preferably an average molar mass of about 200 g/mol.

In one embodiment, the printed ink is annealed at a temperature ranging from 200 to 300°C for 30 minutes to 2 hours. In one embodiment, the ink is a suspension of gold nanoparticles functionalized with said compound Cap in a hydroalcoholic medium.

In one embodiment, steps (a) and (b) are repeated at least twice, preferably steps (a) and (b) are repeated at least four times. In one embodiment, the method further comprises a step of sterilization. In one embodiment, the method further comprises a functionalization step, wherein the gold electrode is functionalized with a biological molecule or a catalyst. In one embodiment, the substrate is organic, inorganic or hybrid. In one embodiment, the substrate is flexible. In one embodiment, the substrate comprises polyimide, polyethylenenaphtalate, glass, silicon dioxide, or a mixture thereof.

The present invention also relates to an electrode obtainable by the method of the invention. In one embodiment, the electrode exhibits a conductivity greater or equal to 1x10⁷ S/m. In one embodiment, the electrode has a thickness ranging from 450 to 550 nm.

The present invention also relates to a sensor comprising at least one electrode according to the invention, wherein said electrode is functionalized with a biological molecule or a catalyst.

The present invention also relates to the use of a sensor according to the invention for detection of glucose, any other diabetes related analytes, lactate, cholesterol, glycated hemoglobin (HbA1c) or a mixture thereof.

### DEFINITIONS

In the present invention, the following terms have the following meanings:
- "About" is used herein to mean approximately, roughly, around, or in the region of. When the term "about" is used in conjunction with a numerical range, it modifies that range by extending the boundaries above and below the numerical values set forth by 5% or 10%.
- "**Alkyl**" refers to any saturated linear or branched hydrocarbon chain, with 1 to 12 carbon atoms, preferably 1 to 6 carbon atoms, and more preferably methyl, ethyl, propyl, isopropyl, n-butyl, sec-butyl, isobutyl and tert-butyl.

### DETAILED DESCRIPTION

The following detailed description will be better understood when read in conjunction with the drawings. For the purpose of illustrating, the device is shown in the preferred embodiments. It should be understood, however that the present invention is not limited to the precise arrangements, structures, features, embodiments, and aspect shown. The drawings are not drawn to scale and are not intended to limit the scope of the claims to the embodiments depicted. Accordingly it should be understood that where features mentioned in the appended claims are followed by reference signs, such signs are included solely for the purpose of enhancing the intelligibility of the claims and are in no way limiting on the scope of the claims.

This invention relates to a method of preparation of a gold electrode.

Said method comprises the steps of:
a) inkjet printing on a substrate an ink comprising gold nanoparticles functionalized with a compound Cap of formula (I)

   HS-A-Polyalkylene glycol (I)

   wherein A is a bound or an alkyl chain comprising 1 to 12 carbon atoms;
   wherein the polyalkylene glycol is polyethylene glycol (PEG), polypropylene glycol (PPG), or a mixture thereof; and
b) annealing printed ink.

The compound Cap has an average molar mass of less than 500 g/mol, preferably an average molar mass of about 200 g/mol.

The compound Cap is a capping agent. Cap absorbs on surface of gold nanoparticles, forming a crown, and ensures good dispersion of gold nanoparticles in ink.

The method of the invention is easy to implement as it only needs an inkjet printer, and provides a precise gold pattern. By gold pattern, it is meant the geometric design of the gold electrode obtained after annealing.

The method of the invention also requires only two steps to provide a gold pattern on a substrate, whereas forming a gold layer by evaporation requires many time-consuming steps, including the masking of the substrate, and has a higher risk of deteriorating the substrate because of the masking steps and treatments of the substrate.

The printed ink is annealed in order to remove, partially or totally, the insulating organic crown formed by the compound Cap. This is especially necessary for applications as a conductive electrode.

Indeed, annealing results in the loss (partial or total) of the organic crown and core-core coalescence between neighboring nanoparticles in the ink providing a continuous conductive surface.

This annealing step should be performed at the lowest possible temperatures to avoid any substrate damages, especially when organic substrates are used as they are low-melting temperature materials. A temperature ranging from 200 to 300°C is low enough to prevent deteriorating the substrate and high enough to remove the compound Cap.

The ink comprises gold nanoparticles functionalized with the compound Cap in a solvent, thus the annealing step also serves to evaporate said solvent.

Gold nanoparticles are prepared by mixing a gold salt, a reducing agent and a phase-transfer agent in a solvent.

According to one embodiment, the compound Cap is mercaptopolyethylene glycol of formula (I) where A is a single bound and where polyalkylene glycol is a polyethylene glycol with average molecular weight of 200 g.mol⁻¹ (*i.e.* an average of 5 repetition units of glycol), noted S-PEG₂₀₀ hereinafter. In this embodiment, S-PEG₂₀₀ increases the hydrophilicity of the gold nanoparticles and allow their dispersion in a hydroalcoholic medium. Thus, it prevents the aggregation of the nanoparticles in the ink.

According to one embodiment, the printed ink is annealed at a temperature ranging from 200 to 300°C for 30 minutes to 2 hours. The low annealing temperature prevents the deterioration of organic substrates. In a preferred embodiment, the printed ink is annealed at 220°C for 1 hour.

According to one embodiment, the ink is a suspension of gold nanoparticles functionalized with said compound Cap in a hydroalcoholic medium. A hydroalcoholic medium refers to a mixture of alcohol, preferably ethanol, and water. In a preferred embodiment, the hydroalcoholic medium is a solution comprising a volume fraction of 60:40 of water and ethanol respectively.

According to one embodiment, the ink has a concentration of gold nanoparticles ranging from 1 to 20 % (w/w), preferably from 1 to 15 % (w/w), preferably from 1 to 10% (w/w), more preferably from 5 to 10% (w/w).

According to one embodiment, the ink has a viscosity ranging from 1 to 50 cP, preferably from 1 to 25 cP, preferably from 1 to 10 cP, more preferably from 1 to 5 cP.

According to one embodiment, the ink exhibits a surface tension ranging from 10 to 72 mN.m⁻¹, preferably from 10 to 60 mN.m⁻¹, preferably from 20 to 50 mN.m⁻¹, more preferably from 30 to 40 mN.m⁻¹.

According to one embodiment, the gold nanoparticles have an average size ranging from 1 to 100 nm, preferably from 1 to 50 nm, preferably from 2 to 25 nm, preferably from 2 to 20 nm, more preferably from 2 to 10 nm, more preferably from 2 to 5 nm.

According to one embodiment, the gold nanoparticles are preferably spherical.

According to one embodiment, the ink is printed using between 5 to 50 µm drop spacing, preferably between 5 to 25 µm drop spacing, more preferably between 5 to 15 µm drop spacing.

According to one embodiment, the ink is printed with 1 to 10 pL-droplet cartridges, preferably the ink is printed with 10 pL-droplet cartridges.

According to one embodiment, the jetting frequency is ranging from 0.5 to 15 kHz, preferably 1 to 10 kHz, more preferably from 1 to 5 kHz.

According to one embodiment, the temperature of the platform of the printer is ranging from 20 to 60°C, preferably from 20 to 50°C, more preferably from 30 to 45°C.

According to one embodiment, the temperature of the cartridge is ranging from 20 to 60°C, preferably from 20 to 50°C, more preferably from 25 to 40°C, more preferably from 25 to 35°C.

According to one embodiment, steps (a) and (b) are repeated at least twice, preferably steps (a) and (b) are repeated at least four times. In this embodiment, the annealing step is performed after each inkjet printing of a single layer, this enables a good evaporation of the solvent for each printed layer. In this embodiment, at least two layers of gold nanoparticles are deposited successively on the substrate, this prevents the formation of holes and cracks in the gold pattern.

According to one embodiment, the method further comprises a functionalization step, wherein the gold electrode is functionalized with a biological molecule or a catalyst.

According to one embodiment, the functionalization step comprises the immersion of the printed electrode in a solution comprising at least one biological molecule, at least one redox active compound or at least one catalyst.

According to one embodiment, examples of biological molecules include but are not limited to: DNA, RNA, antibody, biological molecules comprising at least one sulfur terminal group such as for example thiol group, disulfide group, or dithiolane group. The sulfur group is able to adsorb onto gold surface.

According to one embodiment, examples of redox active compounds include but are not limited to: 6,6'-disulfanediylbis(hexane-6,1-diyl)diferrocenecarboxylate.

According to one embodiment, examples of catalysts include but are not limited to: nanoparticles comprising a thiol functionalization, metal nanoparticles, molecular catalysts, metal alloys, or a mixture thereof.

According to one embodiment, the method further comprises a step of sterilization. In this embodiment, it is possible to provide sterile sensors comprising electrodes prepared according to the method of the invention. This is particularly advantageous for biological applications.

According to one embodiment, the method does not require the use of masks. This is different from the preparation of gold layers by evaporation wherein use of a mask is compulsory to define the desired gold pattern.

According to one embodiment, the obtained gold pattern has sharp edges, i.e. there are no isolated gold clusters on the substrate.

According to one embodiment, the substrate is organic, inorganic or hybrid.

According to one embodiment, the organic substrate comprises a polymer.

According to one embodiment, the substrate is flexible.

According to one embodiment, the substrate comprises polyimide, polyethylenenaphtalate, glass, silicon dioxide, or a mixture thereof.

The invention also relates to an electrode obtainable by the method of the invention.

According to one embodiment, the electrode exhibits a conductivity greater or equal to 1x10⁷ S/m.

According to one embodiment, the electrode has a thickness ranging from 450 to 550 nm.

According to one embodiment, the electrode is a source, drain or gate electrode of a field-effect transistor or an organic electrochemical transistor.

The invention also relates to a sensor comprising at least one electrode of the invention, wherein said electrode is functionalized with a biological molecule or a catalyst.

The invention also relates to a use of a sensor of the invention for detection of glucose, any other diabetes related analytes, lactate, cholesterol, glycated hemoglobin (HbA1c) or a mixture thereof.

According to one embodiment, the gold electrodes obtainable by the method of the invention may be used as interconnects, via contacts, source, drain or gate electrodes of field-effect transistors or of organic electrochemical transistors.

While various embodiments have been described and illustrated, the detailed description is not to be construed as being limited hereto. Various modifications can be made to the embodiments by those skilled in the art without departing from the true spirit and scope of the disclosure as defined by the claims.

### BRIEF DESCRIPTION OF THE DRAWINGS

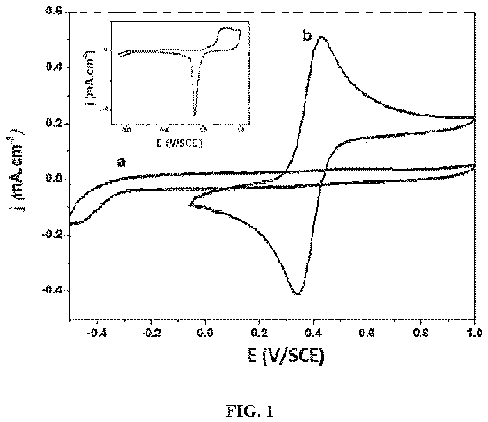

**Figure 1** shows cyclic voltammograms of an electrode comprising 4 gold layers prepared by the method of the invention: (a) in acetonitrile + 0.1 M Tetrabutylammonium hexafluorophosphate (TBAPF₆) as supporting electrolyte and (b) in acetonitrile + 1 mM ferrocene + 0.1 M TBAPF₆ as supporting electrolyte (scan rate: 100 mV/s). Inset: cyclic voltammogram of the electrode at 100 mV/s in 0.5 M H₂SO₄. J (mA.cm⁻²) is the current density, and E (V/SCE) is the electrochemical potential expressed in the unit of Volt/ saturated calomel electrode.

### EXAMPLES

The present invention is further illustrated by the following examples.

### Example 1: Ink formulation

### Gold nanoparticles synthesis

0.225 mmol of HAuCl₄·3H₂O were dissolved in 30 mL of methanol and 5 mL of acetic acid, followed by the addition of 0.1 mmol of compound S-PEG₂₀₀. Once completely dissolved, 2 mmol of freshly prepared NaBH₄ in 5 mL of water were added dropwise. The mixture was stirred for two hours at room temperature. After evaporation of solvent, the nanoparticles were purified by dialysis in deionized water using a 12kDa cut-off membrane and then lyophilized for two days. Said nanoparticles have a size ranging from 2 to 5 nm.

The ink was prepared by suspending the obtained nanoparticles in a mixed solution of deionized water and ethanol (60:40 v/v) to a concentration of 76 mg·mL⁻¹.

### Example 2: Preparation of a gold electrode on a polyimide substrate

A polyimide substrate was cleaned sequentially with acetone, water and isopropanol and was annealed at 220°C for one hour.

The previously obtained ink was printed using a Dimatix Materials Printer (FUJIFILM DIMATIX DMP-2850) with 10 pL-droplet cartridges on the polyimide substrate. The ink was printed using 15 µm drop spacing with jetting frequency of 2 kHz. The platform of the printer and the cartridge temperatures were 40 and 28°C, respectively. The printed ink was then thermally annealed at 220°C for one hour.

### Example 3: Electrode comprising 4 gold layers

The previously obtained ink was printed using a Dimatix Materials Printer (FUJIFILM DIMATIX DMP-2850) with 10 pL-droplet cartridges on the polyimide substrate. The ink was printed using 15 µm drop spacing with jetting frequency of 2 kHz. The platform of the printer and the cartridge temperatures were 40 and 28°C, respectively. The printed ink was then thermally annealed at 220°C for one hour, this resulted in a single layer of S-PEG₂₀₀ functionalized gold nanoparticles on the substrate. A second, third and fourth layers were successively printed and annealed at 220°C for one hour.

### Results

An electrical resistivity of (2.0 ± 0.1) × 10⁻¹ Ω.m and (1 ± 0.1) × 10⁻⁷ Ω.m, i.e. (2.0±0.1) × 10¹ S.m⁻¹ and (1±0.1) × 10⁷ S.m⁻¹ respectively, is obtained for electrodes comprising 2 and 4 layers, respectively.

The thickness of the gold electrodes with 2 and 4 layers, determined by optical profilometry, is respectively 200 and 518 nm.

A sheet resistance of 10⁶ and 0.22 Ω/sq for gold electrodes of 2 and 4 layers, respectively, is estimated. The 4-layers electrode shows extremely low sheet resistance close to that of bulk gold.

Figure 1 shows the electrochemical behavior of a 4-layers electrode in 0.5 M H₂SO₄ medium. A classical gold polycrystalline behavior is observed with the presence of a broad oxidation peak located at 1.3 V and a sharp cathodic peak at 0.8 V/SCE. The cyclic voltammetry performed at different scanning rates in a buffered aqueous solution (PBS) leads to the measure of a differential capacitance of 0.2 mF/cm². This value is greater than what is conventionally encountered in polycrystalline gold due to the high specific printed gold surface area.

Gold electrodes consisting of 4 printed layers exhibited a conductivity of (1±0.1)x10⁷ S/m, close to that of Au bulk, after low-temperature thermal annealing.

### Example 4: Functionalization of an electrode

Two 4-layers electrodes previously obtained were immersed in 6,6'-disulfanediylbis(hexane-6,1-diyl)diferrocenecarboxylate for 35 minutes and 22 hours respectively. The cyclic voltammograms of the electrodes showed the presence of a reversible system corresponding to the ferrocene function, demonstrating the efficient functionalization of the electrodes with ferrocene.

## Claims

1. A method of preparation of a gold electrode comprising the steps of:
c) inkjet printing on a substrate an ink comprising gold nanoparticles functionalized with a compound Cap of formula (I)
HS-A-Polyalkylene glycol (I)
wherein A is a bound or an alkyl chain comprising 1 to 12 carbon atoms;
wherein polyalkylene glycol is polyethylene glycol, polypropylene glycol, or a mixture thereof; and
d) annealing printed ink;
wherein the compound Cap has an average molar mass of less than 500 g/mol, preferably an average molar mass of about 200 g/mol.

2. The method according to claim **1,** wherein the printed ink is annealed at a temperature ranging from 200 to 300°C for 30 minutes to 2 hours.

3. The method according to any one of claim **1** or claim **2,** wherein the ink is a suspension of gold nanoparticles functionalized with said compound Cap in a hydroalcoholic medium.

4. The method according to any one of claims **1** to **3,** wherein steps (a) and (b) are repeated at least twice, preferably steps (a) and (b) are repeated at least four times.

5. The method according to any one of claims **1** to **4,** further comprising a step of sterilization.

6. The method according to any one of claims **1** to **5,** further comprising a functionalization step, wherein the gold electrode is functionalized with a biological molecule or a catalyst.

7. The method according to any one of claims **1** to **6,** wherein the substrate is organic, inorganic or hybrid.

8. The method according to any one of claims **1** to **7,** wherein the substrate is flexible.

9. The method according to any one of claims **1** to **8,** wherein the substrate comprises polyimide, polyethylenenaphtalate, glass, silicon dioxide, or a mixture thereof.

10. An electrode obtainable by the method according to any one of claims **1** to **9.**

11. The electrode according to claim **10,** exhibiting a conductivity greater or equal to 1x10⁷ S/m.

12. The electrode according to claim **11,** having a thickness ranging from 450 to 550 nm.

13. A sensor comprising at least one electrode according to any one of claims **10** to **12,** wherein said electrode is functionalized with a biological molecule or a catalyst.

14. Use of a sensor according to claim **13** for detection of glucose, any other diabetes related analytes, lactate, cholesterol, glycated hemoglobin (HbA1c) or a mixture thereof.
